Europäisches Patentamt

European Patent Office

Office européen des brevets

(19)

(11) Numéro de publication: **0 140 728**
**B1**

## (12) FASCICULE DE BREVET EUROPEEN

(45) Date de publication du fascicule du brevet:
07.01.88

(51) Int. Cl.⁴: **A 61 K 39/395**

(21) Numéro de dépôt: **84401694.9**

(22) Date de dépôt: **21.08.84**

(54) Association pharmaceutique cytotoxique appropriée notamment pour le traitement des cancers.

(30) Priorité: **23.08.83 FR 8313604**

(43) Date de publication de la demande:
**08.05.85 Bulletin 85/19**

(45) Mention de la délivrance du brevet:
**07.01.88 Bulletin 88/1**

(84) Etats contractants désignés:
**BE CH DE FR GB IT LI LU NL SE**

(56) Documents cités:
**EP - A - 0 080 401**

**NATURE, vol. 295, no. 5850, février 1982, pages 604-605, Chesham, Bucks, GB; K.A. KROLICK et al.: "Selective killing of leukaemia cells by antibody-toxin conjugates: implications for autologous bone marrow transplantation"**
**CLINICAL CHEMISTRY, vol. 27, no. 7, juillet 1981, pages 1157-1164, Easton, Pennsylvania, USA; R.F. SCHALL Jr. et al.: "Alternatives to radioimmunoassay: Labels and methods"**

(73) Titulaire: **SANOFI, société anonyme, 40, Avenue George V, F-75008 Paris (FR)**

(72) Inventeur: **Jansen, Franz, Chemin des Fresquets, F-34160 Assas (FR)**
Inventeur: **Gros, Pierre, 18 rue des Mûriers, F-34100 Montpellier (FR)**

(74) Mandataire: **Gillard, Marie-Louise et al, Cabinet Beau de Loménie 55, Rue d'Amsterdam, F-75008 Paris (FR)**

## Description

Dans ses demandes antérieures FR-A-2 437 213 du 28.09.78 et FR-A-2 466 252 du 03.10.79, la demanderesse a décrit la préparation de produits anticancéreux dits conjugués ou encore Immunotoxines obtenus par couplage par liaison covalente de la chaîne A de la ricine à une structure protéique, telle qu'un anticorps, une immunoglobuline ou un fragment d'immunoglobuline, capable de reconnaître sélectivement un antigène donné à la surface des cellules porteuses de cet antigène que l'on veut atteindre, telles que les cellules cancéreuses. La propriété principale de ces conjugués est d'être des agents cytotoxiques spécifiques des cellules cibles visées.

L'utilisation d'anticorps dirigés soit contre des haptènes, soit contre des antigènes de différentiation, soit contre des antigènes associés aux cellules cancéreuses avait déjà permis d'obtenir des conjugués présentant une spécificité remarquable vis-à-vis des cellules cibles et un pouvoir cytotoxique très élevé vis-à-vis de ces mêmes cellules comme cela apparaît dans les demandes de brevets citées ci-dessus ainsi que dans les demandes ultérieures Ep-A-63 988 et Ep-A-80 401 déposées par la demanderesse.

Les conjugués possédant ces propriétés étaient toujours des molécules mixtes artificielles dans lesquelles la chaîne A de la ricine était associée par une liaison covalente de type disulfure à un anticorps, une immunoglobuline ou un fragment d'immunoglobuline capable de reconnaître sélectivement un antigène porté par les cellules cibles visées.

Malgré les remarquables propriétés de cytotoxicité sélective que possèdent ces conjugués à l'égard des cellules portant l'antigène reconnu par l'anticorps constitutif desdits conjugués, il est évident, avec les produits de cette classe, que l'on se trouve devant la nécessité de préparer, d'étudier sur les plans chimique, biologique et médical et de produire pour leur utilisation, autant de conjugués différents que l'on possède d'anticorps dirigés contre des antigènes différents, ou même dirigés contre des épitopes différents du même antigène, susceptibles de servir de cible auxdits conjugués sur les différents types de cellules tumorales que l'on veut détruire.

Poursuivant ses travaux de recherche dans le domaine des conjugués cytotoxiques sélectifs, il est apparu à la demanderesse que cette difficulté pouvait être contournée en utilisant les produits et procédés faisant l'objet de la présente invention.

Selon cette invention, le mécanisme de cytotoxicité spécifique mis en œuvre pour obtenir la destruction sélective des cellules cibles comporte dans sa logique deux étapes:

a) dans une première étape, on met en œuvre un ou plusieurs anticorps ou immunoglobulines ou fragments d'immunoglobulines, possédant la capacité de reconnaissance sélective du ou des antigènes cibles qui ont été choisis pour caractériser les cellules à détruire.

Dans tous les cas, lors de cette première étape, le ou les anticorps ou immunoglobulines ou fragments définis comme indiqué ci-dessus et employés dans leur état naturel, purifiés ou non, sans qu'il soit nécessaire de leur coupler une quelconque substance à propriétés cytotoxiques, sont mis en œuvre dans des conditions leur permettant de se fixer sélectivement sur les cellules à détruire et ainsi de les rendre identifiables en vue de l'étape ultérieure. Si nécessaire l'anticorps non fixé sur les cellules en excès, tout au moins la plus grande partie de cet excès, pourra être éliminé soit par des lavages, soit par toute autre technique appropriée, telle que filtration, centrifugation ou autre technique d'isolement ou de concentration cellulaire,

b) dans une deuxième étape, on met en œuvre au moins un conjugué ou molécule hybride construit en couplant par liaison covalente la chaîne A de la ricine avec un deuxième anticorps ou immunoglobuline ou fragment d'anticorps ou d'immunoglobuline ou encore avec un mélange d'anticorps ou immunoglobulines ou fragments capables de reconnaître sélectivement le ou les premiers anticorps ou immunoglobulines ou fragments, tels que définis et mis en œuvre lors de la première étape. Le conjugué utilisé dans cette deuxième étape est identique aux conjugués déjà décrits et revendiqués dans les précédentes demandes de la demanderesse et les précédents brevets qui lui ont été accordés, excepté que la spécificité choisie pour le ou les anticorps qui entrent dans la constitution de ce ou ces conjugués n'implique pas la reconnaissance sélective d'antigènes présents à la surface des cellules visées et notamment d'antigènes associés à l'état tumoral des cellules mais une spécificité basée sur les critères définis ci-après. Tous les procédés décrits par la demanderesse dans ses brevets et demandes de brevets antérieurs sont applicables à la préparation des conjugués intervenant à la deuxième étape du mécanisme mis en œuvre par la présente invention.

Dans la suite de la présente description et dans les revendications, on désignera par «anticorps» aussi bien les anticorps que les immunoglobulines et les fragments d'immunoglobulines.

La méthodologie générale permettant d'obtenir l'expression d'une cytotoxicité spécifique, telle qu'elle vient d'être décrite, comporte dans son aspect logique deux étapes qui ont été nettement distinguées pour faciliter la compréhension du mécanisme impliqué. En pratique, selon l'invention, la mise en œuvre de ce mécanisme pourra néanmoins avoir lieu:

— soit effectivement en deux étapes, chronologiquement distinctes, comme cela a été décrit ci-dessus,

— soit en utilisant simultanément le ou les premiers anticorps d'une part et le ou les conjugués cytotoxiques d'autre part, tous ces produits étant mis en présence des cellules cibles en une seule fois,

— soit enfin en utilisant des complexes préformés entre le ou les premiers anticorps et le ou les conjugués cytotoxiques, lesdits complexes étant préparés et éventuellement purifiés avant leur mise en présence des cellules cibles. L'avantage de ce procédé est alors d'éviter à la fois tout excès du ou des premiers anticorps, toute opération d'élimination de cet excès et tout excès du ou des conjugués cytotoxiques.

Bien que, dans ce dernier cas, l'ordre chronologique de mise en œuvre des produits soit différent de celui adopté dans la description précédente, nous continuerons dans la suite du texte à désigner par «premiers anticorps» ou «anticorps utilisés lors de la première étape» ceux dont la spécificité correspond à la reconnaissance d'un ou plusieurs antigènes des cellules cibles.

Les critères retenus pour définir la spécificité du ou des anticorps constitutifs du conjugué cytotoxique mis en œuvre à la deuxième étape sont, selon l'invention, fondamentalement liés au fait que le ou les anticorps utilisés à la première étape sont toujours un ou des anticorps caractéristiques d'une espèce animale différente de celle des cellules cibles. Il en résulte qu'il suffit que le ou les anticorps constitutifs du conjugué cytotoxique soient spécifiques des immunoglobulines de l'espèce animale à laquelle appartiennent le ou les anticorps utilisés lors de la première étape pour que le conjugué cytotoxique se fixe aux seules cellules ayant, au cours de la première étape, déjà fixé le ou les premiers anticorps. Ce critère de spécificité d'espèce est généralement suffisant si le ou les anticorps du conjugué sont des anticorps de nature polyclonale. Il en est de même si le ou les anticorps du conjugué sont des immunoglobulines polyclonales, capables de reconnaître toutes les immunoglobulines de l'espèce animale à laquelle appartiennent le ou les anticorps utilisés lors de la première étape. On peut aussi utiliser alternativement des immunoglobulines monoclonales ayant la capacité de reconnaître sélectivement la classe d'immunoglobulines (A, D, E, G, M en particulier) à laquelle appartient le ou les premiers anticorps en fonction de la nature des chaînes lourdes constitutives des immunoglobulines de cette classe dans l'espèce déterminée (par exemple, les chaînes alpha, delta, epsilon, gamma ou mu) ou en fonction de la nature des chaînes légères constitutives des immunoglobulines dans l'espèce déterminée (par exemple, les chaînes kappa ou lambda), ou encore ayant la capacité à reconnaître sélectivement la ou les sous-classes (ou isotypes) du ou des premiers anticorps dans l'espèce déterminée.

Ainsi, à titre d'exemple non limitatif, si le premier anticorps utilisé est une immunoglobuline de classe G et d'isotype 2a de souris (IgG 2a de souris) à chaîne lourde de type gamma et chaîne légère kappa appliquée sur des cellules d'origine humaine, l'anticorps utilisé pour la construction du conjugué cytotoxique peut être préparé à partir d'immunoglobulines polyclonales ou monoclonales anti-immunoglobulines totales de souris ou anti-IgG totales de souris, ou antichaînes gamma d'immunoglobulines de souris, ou anti-chaînes gamma d'immunoglobuliens de souris, ou antichaînes kappa d'immunoglobulines de souris ou anti-IgG 2a de souris, produites par les cellules spécialisées productrices d'anticorps de n'importe quelle espèce convenable pour ce but.

Un raisonnement analogue s'appliquerait quels que soient l'espèce, la classe et éventuellement l'isotype du ou des premiers anticorps utilisés.

Selon la présente invention, les anticorps ou immunoglobulines ou fragments d'anticorps ou d'immunoglobulines utilisés soit à la première étape, soit pour la préparation du conjugué cytotoxique utilisé à la deuxième étape du processus peuvent être indifféremment:

– soit de nature polyclonale, s'ils proviennent du sang d'un animal préalablement immunisé par les méthodes conventionnelles en utilisant un immunogène porteur de l'antigène cible choisi,

– soit de nature monoclonale, s'ils sont produits par des cellules hybrides et notamment par des hybridomes, ces cellules hybrides étant elles-mêmes obtenues en fusionnant des cellules spléniques d'animal immunisé contre l'antigène choisi avec des cellules par exemple de myélome, selon les procédés bien connus de l'homme de l'art. Dans ce cas, l'étape d'immunisation peut être réalisée soit in vivo par administration convenable de l'immunogène chez l'animal à immuniser, soit in vitro par mise en contact direct des cellules spléniques avec l'immunogène (voir en particulier LUBEN R.A., MOHLEP M.A., NEDWIN G.E., Glin. Invest. 64, § 7 (1979) ). En outre, l'anticorps recherché pourra être obtenu et purifié ou isolé, si cela est nécessaire, soit à partir de surnageants de culture des cellules hybrides, soit à partir du sang ou du liquide d'ascite d'animaux inoculés avec lesdites cellules hybrides.

Il apparaît clairement que, dans un tel système comportant dans sa logique deux étapes, la sélectivité globale du système est assurée par le ou les anticorps utilisés à la première étape qui repèrent les cellules porteuses du ou des antigènes cibles choisis et elles seules. L'effet cytotoxique est dû au conjugué mis en œuvre au cours de la deuxième étape et cet effet cytotoxique ne concerne que les cellules ayant fixé le ou les premiers anticorps, en raison des critères énoncés ci-dessus pour le choix du ou des anticorps constitutifs du conjugué cytotoxique utilisé à la deuxième étape.

En bref, le fonctionnement général de ce système est l'homologue de celui de la technique d'immunofluorescence indirecte bien connue des cytologistes, à cette différence près que, dans l'invention, le deuxième anticorps porte la chaîne A de ricine comme agent cytotoxique puissant capable de tuer les cellules repérées par le ou les premiers anticorps alors que, dans la technique d'immunofluorescence indirecte, le deuxième anticorps porte seulement un composé fluorescent permettant de visualiser les cellules repérées par le premier anticorps.

Un premier avantage d'un tel système apparaît immédiatement puisqu'il suffit de disposer d'un seul ou en tout cas d'un petit nombre de conjugués correspondant aux combinaisons les plus fréquentes des critères d'espèces, de classes et d'isotypes indiqués précédemment pour rendre cytotoxiques des anticorps non modifiés, en nombre pratiquement illimité, dirigés contre une très grande variété d'antigènes membranaires des cellules à détruire. Cette propriété permet d'amplifier de façon considérable le champ d'application des conjugués cytotoxiques résultant du couplage de la chaîne A de ricine avec un anticorps.

Une deuxième propriété avantageuse de ces système à deux étapes est qu'ils manifestent des propriétés de cytotoxicité sélective tout à fait analogues et même parfois supérieures à celles des systèmes antérieurement décrits et comportant une seule étape. Une telle analogie de comportement se vérifie d'une part au niveau des concentrations de conjugué cytotoxique à employer pour obtenir un effet cytotoxique fixé à l'avance (par exemple 50% de destruction de la population des cellules cibles) et d'autre part au niveau de la cinétique de cytotoxicité, c'est-à-dire du temps nécessaire pour qu'une fraction fixée à l'avance de la population de cellules cibles soit détruite.

Une troisième propriété avantageuse des systèmes à deux étapes est qu'ils sont potentialisables par les mêmes substances et dans les mêmes conditions que les systèmes antérieurement décrits et comportant une seule étape. Il en résulte que toutes les substances potentialisatrices des immunotoxines déjà revendiquées par la demanderesse dans ses demandes antérieures Ep-A-86 152, Ep-A-89 270, Ep-A-88 694 et Ep-A-89 880 peuvent aussi bien être utilisées dans les systèmes à deux étapes, objet de la présente invention, avec tous les avantages au niveau de l'efficacité cytotoxique de la cinétique de cytotoxicité et de la spécificité qui ont déjà été préalablement démontrés avec les immunotoxines précédemment décrites.

Un quatrième avantage des systèmes à deux étapes est qu'ils permettent avec un seul ou un petit nombre de conjugués cytotoxiques employés lors de la deuxième étape d'exploiter la fixation cumulée et simultannée, lors de la première étape, de plusieurs anticorps non modifiés sur les mêmes cellules cibles. Ceci se traduit toujours par un gain important d'efficacité cytotoxique, comme cela est démontré dans les exemples décrits. Cette propriété est particulièrement intéressante lorsque les cellules tumorales ne présentent pour aucun des antigènes cibles utilisables pris isolément des densités d'expression à la membrane cellulaire suffisamment élevées pour que l'efficacité cytotoxique soit satisfaisante. Dans les systèmes à une seule étape, cette difficulté ne peut être surmontée qu'en utilisant simultanément plusieurs conjugués cytotoxiques construits chacun avec un anticorps dirigé contre l'un des antigènes cibles possibles. Toutefois, cela se traduit par une augmentation globale de la concentration des produits cytotoxiques dans le milieu et l'on risque alors d'atteindre une concentration totale à laquelle les effets secondaires sur les cellules non cibles ne sont plus négligeables. Par contre, dans un système à deux étapes, on dispose de la possibilité d'utiliser simultanément ou séquentiellement à la première étape plusieurs anticorps non modifiés dirigés chacun contre un antigène cible possible, ce qui permet d'additionner les densités individuelles de ces antigènes. Comme la toxicité non spécifique des anticorps non modifiés n'est jamais en pratique un facteur limitant et que, de toute façon, l'excès de ces anticorps peut être facilement éliminé, les risques à l'égard des cellules non cibles sont alors négligeables à cette étape. Ensuite, à la deuxième étape, un seul conjugué cytotoxique peut être utilisé, à une concentration qui n'est pas différente selon qu'à la première étape on a utilisé un ou plusieurs anticorps non modifiés. Il en résulte donc que le risque de toxicité non spécifique n'est pas non plus accru lors de la deuxième étape.

En outre, un avantage supplémentaire lié à la possibilité de mettre en œuvre dans les systèmes à deux étapes plusieurs antigènes différents portés en commun par les cellules cibles, sans que cela implique une augmentation du risque de toxicité non spécifique à l'égard des cellules non cibles, est d'augmenter le caractère spécifique de la cytotoxicité à l'égard des cellules cibles. En effet, même si les antigènes choisis ne sont pas strictement spécifiques de la population des cellules cibles, mais peuvent être individuellement exprimés aussi par des cellules non cibles, la probabilité que plusieurs de ces antigènes, s'ils sont bien choisis, soient simultanément présents sur des cellules non cibles est très faible et l'on dispose ainsi d'un moyen puissant pour augmenter la sélectivité du système cytotoxique.

Enfin, lorsque, en pratique, les systèmes à deux étapes sont utilisés en respectant la séparation chronologique entre les deux étapes et en procédant à l'élimination de l'excès du ou des premiers anticorps, une propriété avantageuse supplémentaire apparaît du fait que le ou les premiers anticorps employés peuvent n'être que des anticorps purifiés. En effet, la procédure d'élimination de l'excès d'anticorps éliminera aussi tous les autres produits constituant les impuretés de la préparation. Ainsi, par exemple, des sérums d'animaux immunisés, fractionnés ou non, des surnageants de culture d'hybridome, des liquides d'ascite d'animaux inoculés avec les cellules d'hybridome, peuvent être utilisés directement sans purification préalable très poussée.

Les systèmes cytotoxiques sélectifs à deux étapes, objets de la présente invention, peuvent être utilisés dans toutes les situations dans lesquelles pouvaient déjà être utilisés les conjugués cytotoxiques sélectifs ou immunotoxines, fonctionnant en une seule étape. En outre, ces systèmes à deux étapes peuvent être utilisés conjointement dans chacune de ces situations avec n'importe lequel des systèmes de potentialisation ou des produits potentialisateurs déjà décrits antérieurement par la demanderesse. Les principaux types de situations où l'utilisation de tels systèmes est particulièrement valable sont:

a) chaque fois qu'un système ou un agent cytotoxique sélectif est utile in vitro pour la destruction spécifique d'une certaine population cellulaire en présence d'autres cellules à préserver. En dehors de nombreuses utilisations possibles en laboratoire dans des travaux de recherche, cette situation se rencontre aussi dans le domaine thérapeutique, notamment lorsque de tels systèmes ou agents cytotoxiques sélectifs sont utilisés dans le traitement de la moelle osseuse de patients cancéreux et, notamment leucémiques, à qui la moelle ainsi traitée, afin d'obtenir la diminution ou l'éradication de la population cellulaire tumorale, sera ultérieurement retransplantée (protocole dit d'autogreffe médullaire).

Une situation analogue se présente dans les protocoles de greffe médullaire allogénique. Dans ce cas, la population cellulaire à détruire sélectivement n'est pas tumorale mais est représentée par les lymphocytes T matures du donneur qui sont responsables chez le receveur de la maladie dite du «greffon contre l'hôte» s'ils ne sont pas supprimés dans les cas d'histocompatibilité incomplète entre donneur et receveur,

b) chaque fois qu'un système ou agent cytotoxique sélectif est utile in vivo en tant qu'agent thérapeutique pour l'élimination des cellules indésirables, notamment tumorales, qu'elles appartiennent à la tumeur primaire ou à des métastases.

Les exemples suivants permettent de mieux comprendre l'invention sans en limiter la portée.

Exemple 1

Le modèle cellulaire utilisé dans cet exemple est constitué par des cellules de la lignée lymphoblastoïde humaine CEM, issue d'une leucémie lymphoblastique T et met en œuvre trois antigènes cibles différents, tous trois exprimés par cette lignée, et qui sont les suivants:

— antigène T-200, commun aux leucocytes humains, reconnu par l'anticorps monoclonal T 29–33 (voir notamment H. BATTOFORA et I. TROWBRIDGE Cancer 51 (1983) 816–821),

— antigène T-65, commun aux lymphocytes T humains, reconnu par l'anticorps monoclonal T 101 (voir notamment S.B. WORMSLEY, M.L. COLLINS et I. ROYSTON, Blood 57 (1981) 657–662),

— antigène du système HLA, reconnu par l'anticorps monoclonal MAS-015b (commercialisé par SERA LAB.).

Les anticorps monoclonaux non modifiés désignés ci-dessus sont ceux qui ont été utilisés, isolément ou en combinaison en première étape du système à deux étapes.

En deuxième étape, a été utilisé un conjugué cytotoxique unique construit par couplage covalent à l'aide d'un bras à pont disulfure entre la chaîne A de ricine d'une part et une préparation d'immunoglobulines polyclonales anti-IgG de souris obtenues chez le mouton immunisé par injections intradermiques et intramusculaires d'IgG pures de souris et purifiées par chromatographie d'affinité.

Ce conjugué cytotoxique a été obtenus selon une technique analogue á celles décrties dans nos demandes antérieures FR-A-2 437 213 et FR-A-2 465 252, Ep-A-63 988 et Ep-A-80 401. Pour cela, 11 ml de la solution d'immunoglobulines purifiées de mouton anti-IgG de souris, soit 50 mg d'anticorps, sont additionnées de 0,2 ml de la solution obtenue en mélangeant 5 volumes de solution à 20 mg/ml d'acide (pyridyl-2 disulfanyl)-3 propionique dans le butanol tertiaire et 1 volume de solution aqueuse à 60 mg/ml d'éthyl-1 (diéthylamino-3 propyl)-3 carbodiimide. Ce mélange est incubé 20 h à 30°C puis dialysé en continu à 4 °C pendant 48 h contre au total 40 l de tampon phosphate 0,125 M pH 7,0. Après centrifugation on obtient 11,5 ml de solution d'IgG modifiée à 3,6 mg/ml, le taux de modification étant de 3,0 groupements dithiopyridyl introduits par mole d'immunoglobuline. 11 ml de cette solution d'anticorps activés sont mélangés à 3,1 ml de solution de chaîne A de ricine à 8,9 mg/ml. Le mélange est incubé pendant 20 h à 25 °C, centrifugé puis chromatographié sur une colonne de Sephadex G100 de diamètre 2,6 cm et de hauteur 100 cm. Les fractions correspondant aux molécules de poids moléculaires supérieurs ou égaux à 150 000 sont rassemblées et conduisent à 57 ml d'une solution de conjugué cytotoxique (soit 33 mg) contenant en moyenne 1,5 molécule de chaîne A de ricine couplée par molécule d'immunoglobuline.

A titre de contrôle, ont été également utilisés dans ces expériences un conjugué cytotoxique à spécificité anti-T65, indentique à celui qui a été décrite dans l'exemple 1 de la demande précédente Ep-A-80 401 de la demanderesse, et un conjugué cytotoxique à spécificité anti-T-200, construit à l'aide de l'anticorps T29−33 dans les mêmes conditions de préparation que le conjugué anti-T-65.

A. Efficacité cytotoxique et spécificité du système à deux étapes:

Six lots identiques de cellules CEM maintenues en milieu RPMI 1640 contenant 10% de sérum foetal de veau (milieu de culture) à raison de $5.10^5$ cellules/ml sont respectivement additionnés comme indiqué ci-dessous:

lot 1: rien

lot 2: anticorps T29−33 (anti-T-200) à 30 µg/ml final

lot 3: anticorps T101 (anti-T65) à 30 µg/ml final lot 4: anticorps MAS-015b (anti-HLA) à 30 µg/ml final

lot 5: rien

lot 6: rien

et incubés pendant 2 h à 4°C. Après lavages répétés 3 fois à 4°C à l'aide de milieu de culture, les cellules des 6 lots sont mises en présence de chlorure d'ammonium à la concentration finale de 10 mM (comme potentialisateur). Dans les 4 premiers lots est ajouté le conjugué cytotoxique anti-Immunoglobines G de souris à la concentration finale de $5.10^{-8}$ M, exprimée en chaîne de ricine.

Dans les 5e et 6e lots, sont ajoutés de même respectivement le conjugué cytotoxique anti-T-65 et le conjugué cytotoxique anti-T-200 aux mêmes concentrations finales de $5.10^{-8}$ M exprimées en chaîne A de ricine.

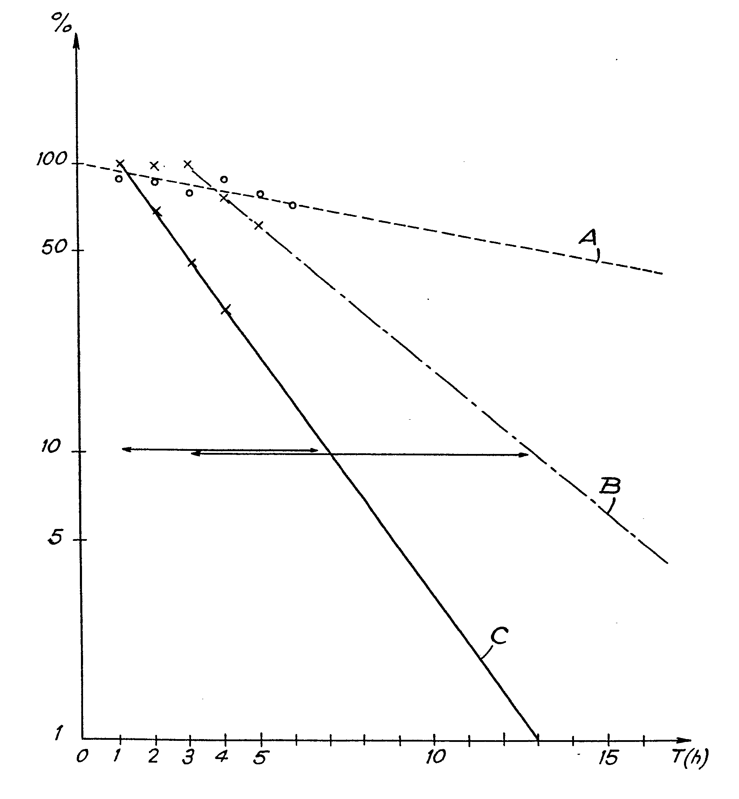

A partir de ce stade, les 6 lots de cellules sont incubés dans les mêmes conditions à 37 °C. A différents temps compris entre 0 et 30 h après la mise en contact des cellules et du conjugué cytotoxique, des aliquotes appartenant à chacun des lots de cellules sont soumises au test de mesure de la cytotoxicité. Ce test consiste à mesurer la capacité des cellules à incorporer de la $^{14}$C-leucine, selon une technique adaptée de celle décrite dans J. of Biol. Chem. 249 (1974) 3557–62. La détermination de la radioactivité incorporée est effectuée ici sur les cellules entières isolées par filtration. La radioactivité incorporée est calculée en pourcentage de la valeur trouvée pour les cellules-témoins incubées dans les mêmes conditions mais en l'absence de tout agent cytotoxique. Les résultats obtenus sont représentés en fonction du temps d'incubation des cellules avec le conjugué cytotoxique, en coordonnées semi-logarithmiques, comme le montre la figure I à titre d'exemple pour les lots 1–3 et 5.

Sur cette figure, on a porté en abscisses le temps d'incubation à 37 °C en heures et en ordonnées le pourcentage de protéosynthèse cellulaire en échelle logarithmique. Les courbes A, B, C correspondent respectivement aux lots ci-après:
courbe A : lot 1, conjugué anti-IgG de souris seul,
courbe B : lot 3, anticorps anti-T-65 + conjugué anti-IgG seul,
courbe C : lot 5, conjugué anti-T-65 seul.

A partir de ces résultats, on peut aussi calculer le temps d'incubation nécessaire pour que le taux résiduel d'incorporation du traceur soit égal au dixième de la valeur initiale des cellules témoins. Ce temps désigné par le symbole T10 et exprimé en heures figure dans le tableau 1 qui récapitule l'ensemble de l'expérience. Il est à noter que ces valeurs de T10 sont corrigées pour la durée de la période initiale de latence pendant laquelle aucune inhibition d'incorporation n'est décelable.

Ces résultats permettent de tirer les conclusions suivantes:
— Avec chacun des 3 anticorps mis en œuvre en première étape (lots 2–3 et 4), le système à deux étapes conduit à une cytotoxicité très élevée, puisque, dans tous les cas après 30 h, l'incorporation du traceur n'est plus détectable.
— Cet effet cytotoxique est sous la stricte dépendance de la présence de l'anticorps utilisé dans la première étape car, en son absence (lot 1), le même conjugué cytotoxique anti-immunoglobulines de souris qui ne trouve pas de cible sur les cellules n'induit aucune cytotoxicité.
— Chaque fois que l'on peut comparer la cytotoxicité obtenue grâce à un conjugué cytotoxique utilisé seul avec celle obtenue grâce au système à deux étapes impliquant en première étape le même anticorps que celui qui entre dans la constitution du conjugué cytotoxique précédent, on observe des cinétiques relativement similaires indiquant des efficacités cytotoxiques du même ordre de grandeur. Il est toutefois intéressant de noter que dans un cas (antigène T65, comparaison lot 3 versus lot 5) le système à deux étapes est un peu moins efficace que le conjugué cytotoxique seul, alors que, pour l'antigène T-200 (comparaison lot 2 versus lot 6), le système à deux étapes est plus efficace que le conjugué cytotoxique seul.

B. Intérêt de l'utilisation de plusieurs anticorps à la première étape:
Dans des conditions tout à fait analogues à celles décrites dans le paragraphe A ci-dessus, 8 lots de cellules CEM ont été mis en œuvre. Le tableau 2 rassemble, comparativement au tableau 1, les traitements appliqués aux 8 lots utilisés. Il apparaît qu'à la première étape du système à deux étapes les 3 anticorps précédemment désignés ont été mis en œuvre soit isolément, soit groupés deux par deux (selon les 3 combinaisons possibles), soit groupés les trois ensemble. Dans tous les cas, chaque anticorps a été utilisé à la concentration de 30 µg/ml, qu'il soit seul ou associé à d'autres, et le conjugué cytotoxique a été employé à la concentration finale de $5.10^{-8}$ M exprimée en chaîne A de ricine, quel qu'ait été le nombre d'anticorps employés à la première étape.

Les résultats de cytotoxicité exprimés en T10 (en heures) figurent sur le tableau 2 et permettent de tirer les conclusions suivantes:
— les lots 1, 2, 3 et 4 qui sont une répétition de l'expérience précédente donnent des résultats parfaitement cohérents avec ceux donnés dans le tableau 1,
— lorsque les anticorps non modifiés utilisés à la première étape sont combinés deux par deux, un gain de cytotoxicité est observé dans chaque cas:
● lot 5 versus lots 2 et 3 : 6,5 h contre 15 et 10 h.
● lot 6 versus lots 3 et 4 : 6,0 h contre 10 et 6,7 h.
● lot 7 versus lots 2 et 4 : 5,3 h contre 15 et 6,7 h.
— lorsque enfin les anticorps non modifiés sont utilisés tous les trois ensemble, l'efficacité cytotoxique maximale est obtenue (T10 = 5 h),
— ces résultats démontrent très clairement l'accélératon de la cinétique de cytotoxicité que l'on peut obtenir lorsque l'on met en œuvre simultanément plusieurs antigènes membranaires, sans qu'il soit nécessaire d'augmenter la concentration du conjugué cytotoxique utilisé. Ce résultat est de la plus haute importance pour toutes applications thérapeutiques dans lesquelles on doit évidemment chercher à obtenir la meilleure efficacité cytotoxique possible, dans le délai le plus court et en mettant en œuvre la plus petite concentration possible de conjugué cytotoxique.

Comme il apparaît clairement des résultats de l'exemple ci-dessus, les produits et procédés — objet de la présente invention — constituent, lorsqu'ils sont mis en œuvre dans un but thérapeutique, une association de principes actifs dans laquelle aucun des principes actifs utilisé seul ne présente d'activité significative, alors que l'emploi combiné de représentants de chacune des deux classes de principes actifs concernés fait apparaître un très haut niveau d'activité cytotoxique d'intérêt thérapeutique.

Selon les modalités pratiques d'emploi thérapeutique souhaitées et en fonction des indications précises du traitement, les produits, objet de l'invention, seront utilisés sous forme de médicaments selon deux grands types de présentations possibles:

– dans la présentation du premier type, le ou les anticorps non modifiés seront présentés sous la forme de solutés injectables contenant chaque anticorps séparément ou un mélange des différents anticorps à utiliser. Chaque flacon unitaire contiendra, de préférence, entre 0,5 et 20 mg de chaque anticorps dans un véhicule aqueux, isotonique et tamponné à une valeur de pH comprise de préférence entre 4 et 9. Le conditionnement réalisé permettra soit une administration intraveineuse directe, soit l'introduction dans un dispositif de perfusion intraveineuse, soit l'introduction dans les récipients contenant les échantillons biologiques à traiter, notamment les échantillons de moelle osseuse.

En outre, cette présentation comprendra le ou les conjugués cytotoxiques sous la forme de solutés injectables contenant chaque conjugué séparément ou un mélange des conjugués nécessaires. Chaque flacon contiendra, de préférence, de 0,5 à 20 mg de chaque conjugué dans un véhicule et selon un conditionnement analogues à ceux décrits ci-dessus.

La présentation de ce premier type permet le maximum de souplesse d'utilisation des produits de l'invention dans la mesure où elle laisse au thérapeute toute latitude d'utiliser le ou les anticorps non modifiés et le ou les conjugués cytotoxiques dans l'ordre chronologique le plus adapté à l'application visée:

– dans la présentation du deuxième type, le ou les produits à utiliser sont constitués par des solutés injectables contenant le ou les complexes solubles préformés entre le ou les anticorps non modifiés et le ou les conjugués cytotoxiques. Dans ces préparations, les constituants assurant la formation des complexes sont présents dans un rapport molaire de préférence compris entre 0,1 et 10 et choisis dans chaque cas particulier, de telle sorte que la préparation reste parfaitement limpide. Les complexes solubles ainsi formés peuvent être soit utilisés tels quels, c'est-à-dire en présence de l'excès de celui des constituants qui n'est pas employé dans la complexation, soit être préalablement purifiés, par exemple par filtration sur gel, pour éliminer tout ou une partie de cet excès. Dans tous les cas, ces produits sont présentés par flacons unitaires contenant, de préférence, de 0,5 à 20 mg de produit utile, dans un véhicule et selon un conditionnement analogues à ceux décrits ci-dessus.

Tableau 1

| Lot n° | 1ère étape (anticorps non modifié) | 2e étape (conjugué cytotoxique) | $T_{10}$ (heures) |
|---|---|---|---|
| 1 | – | conjugué anti-IgG de souris | $\infty$ |
| 2 | anticorps T29–33 (anti-T200) | id | 6,5 |
| 3 | anticorps T101 (anti-T-65) | id | 10 |
| 4 | anticorps MAS–015b (anti-HLA) | id | 15 |
| 5 | – | conjugué anti-T65 | 6,0 |
| 6 | – | conjugué anti-T200 | 15 |

Tableau 2

| Lot n° | 1ère étape (anticorps non modifié) | 2e étape (conjugué cytotoxique) | $T_{10}$ (heures) |
|---|---|---|---|
| 1 | | Conjugué anti-IgG de souris | $\infty$ |
| 2 | anti-HLA | id | 15 |
| 3 | anti-T-65 | id | 10 |
| 4 | anti-T-200 | id | 6,7 |
| 5 | anti-T-65 + anti-HLA | id | 6,5 |
| 6 | anti-T-200 + anti-T-65 | id | 6,0 |
| 7 | anti-T-200 + anti-HLA | id | 5,3 |
| 8 | anti-HLA + anti-T-65 + anti-T-200 | id | 5,0 |

**Revendications**

1. Association pharmaceutique cytotoxique, caractérisée en ce qu'elle comprend,

– d'une part, au moins un premier anticorps possédant la capacité de reconnaissance sélective de ou des antigènes cibles portés par les cellules à détruire, ledit anticorps étant caractéristique d'une espèce animale différente de celle des cellules cibles;

– d'autre part, au moins un conjugué cytotoxique obtenu par couplage par liaison covalente de la chaîne A de la ricine avec un second anticorps spécifique des immunoglobulines de l'espèce animale à laquelle appartiennent le ou lesdits premiers anticorps.

2. Association pharmaceutique cytotoxique selon la revendication 1, caractérisée en ce que ledit premier anticorps et ledit conjugué cytotoxique sont dans des conditionnements distincts.

3. Association pharmaceutique cytotoxique selon l'une des revendications 1 ou 2, caractérisée en ce que ledit premier anticorps et ledit conjugué cytotoxique sont chacun sous la forme d'un soluté injectable.

4. Association pharmaceutique cytotoxique selon l'une quelconque des revendications 1 à 3, caractérisée en ce que lesdits premiers anticorps sont dans des solutés injectables distincts.

5. Association pharmaceutique cytotoxique se-

lon l'une quelconque des revendications 1 à 3, caractérisée en ce que lesdits premiers anticorps sont dans le même soluté injectable.

6. Association pharmaceutique cytotoxique selon l'une quelconque des revendications 1 à 5, caractérisée en ce que lesdits conjugués cytotoxiques sont dans des solutés injectables distincts.

7. Association pharmaceutique cytotoxique selon l'une quelconque des revendications 1 à 5, caractérisée en ce que lesdits conjugués cytotoxiques sont dans le même soluté injectable.

8. Association pharmaceutique cytotoxique selon l'une quelconque des revendications 1 à 7, caractérisée en ce que chaque conditionnement représente une dose unitaire contenant 0,5 à 20 mg dudit premier anticorps ou dudit conjugué cytotoxique.

9. Association pharmaceutique cytotoxique selon la revendication 1, caractérisée en ce qu'elle est constituée d'un soluté injectable contenant à la fois ledit premier anticorps et ledit conjugué cytotoxique dans un rapport molaire compris entre 0,1 et 10.

10. Association pharmaceutique cytotoxique selon la revendication 1, utile pour le traitement des cancers, caractérisée en ce que ledit premier anticorps est un anticorps anticellules tumorales.

11. Association pharmaceutique cytotoxique selon la revendication 10, caractérisée en ce que ledit premier anticorps est choisi parmi les anticorps anti-T65, anticorps anti-T200, anticorps anti-HLA et en ce que ledit conjugué cytotoxique est le produit de couplage de la chaîne A de la ricine avec des IgC de souris.

## Patentansprüche

1. Cytotoxische pharmazeutische Assoziation, dadurch gekennzeichnet, dass sie enthält:

– einerseits mindestens einen ersten Antikörper, der befähigt ist, einen oder mehrere von den zu zerstörenden Zellen getragene Targetantikörper selektiv zu erkennen, und für eine Art von Lebewesen charakteristisch ist, die von derjenigen der Targetzellen verschieden ist, und

– andererseits mindestens ein cytotoxisches Konjugat, das durch kovalente Verknüpfung der A-Kette des Ricins mit einem zweiten spezifischen Antikörper der Immunglobuline derjenigen Art von Lebewesen erhalten ist, zu welcher der bzw. die ersten Antikörper gehören.

2. Cytotoxische pharmazeutische Assoziation nach Anspruch 1, dadurch gekennzeichnet, dass der erste Antikörper und das cytotoxische Konjugat in unterschiedlichen Packungen vorliegen.

3. Cytotoxische pharmazeutische Assoziation nach Anspruch 1 oder 2, dadurch gekennzeichnet, dass der erste Antikörper und das cytotoxische Konjugat jeweils in Form einer Injektionslösung vorliegen.

4. Cytotoxische pharmazeutische Assoziation nach einem der Ansprüche 1 bis 3, dadurch gekennzeichnet, dass die ersten Antikörper in unterschiedlichen Injektionslösungen vorliegen.

5. Cytotoxische pharmazeutische Assoziation nach einem der Ansprüche 1 bis 3, dadurch gekennzeichnet, dass die ersten Antikörper in derselben Injektionslösung vorliegen.

6. Cytotoxische pharmazeutische Assoziation nach einem der Ansprüche 1 bis 5, dadurch gekennzeichnet, dass die cytotoxischen Konjugate in unterschiedlichen Injektionslösungen vorliegen.

7. Cytotoxische pharmazeutische Assoziation nach einem der Ansprüche 1 bis 5, dadurch gekennzeichnet, dass die cytotoxischen Konjugate in derselben Injektionslösung vorliegen.

8. Cytotoxische pharmazeutische Assoziation nach einem der Ansprüche 1 bis 7, dadurch gekennzeichnet, dass jede Packung eine Einheitsdosis darstellt, die 0,5 bis 20 mg des ersten Antikörpers oder des cytotoxischen Konjugats enthält.

9. Cytotoxische pharmazeutische Assoziation nach Anspruch 1, dadurch gekennzeichnet, dass sie aus einer Injektionslösung besteht, die sowohl den ersten Antikörper als auch das cytotoxische Konjugat in einem Molverhältnis zwischen 0,1 und 10 enthält.

10. Cytotoxische pharmazeutische Assoziation nach Anspruch 1, zur Krebsbehandlung, dadurch gekennzeichnet, dass der erste Antikörper ein Antitumorzellen-Antikörper ist.

11. Cytotoxische pharmazeutische Assoziation nach Anspruch 10, dadurch gekennzeichnet, dass der erste Antikörper unter Anti-T65-, Anti-T200- und Anti-HLA-Antikörpern ausgewählt ist, und das cytotoxische Konjugat das Produkt der Verknüpfung der A-Kette des Ricins mit Mäuse-IgG darstellt.

## Claims

1. Cytotoxic pharmaceutical combination, characterized in that it comprises,

– on the one hand, at least a first antibody possessing the capacity of selective recognition of a target antigen or antigens carried by cells to be destroyed, the said antibody being characteristic of a different animal species from that of the target cells,

– on the other hand, at least one cytotoxic conjugate obtained by covalent bond coupling of the A chain of ricin with a second specific antibody of immunoglobins of the animal species to which the said first antibody or antibodies belong.

2. Cytotoxic pharmaceutical combination according to claim 1, characterized in that said first antibody and said cytotoxic conjugate are in separate packages.

3. Cytotoxic pharmaceutical combination according to any one of claims 1 or 2, characterized in that said first antibody and said cytotoxic conjugate are each in the form of an injectable solution.

4. Cytotoxic pharmaceutical combination according to anyone of claims 1 to 3, characterized in that said first antibodies are in distinct injectable solutions.

5. Cytotoxic pharmaceutical combination according to any one of claims 1 to 3, characterized in that said first antibodies are in the same injectable solution.

6. Cytotoxic pharmaceutical combination according to any one of claims 1 to 5, characterized in that said cytotoxic conjugates are in distinct injectable solutions.

7. Cytotoxic pharmaceutical combination according to any one of claims 1 to 5, characterized in that said cytotoxic conjugates are in the same injectable solution.

8. Cytotoxic pharmaceutical combination according to any one of claims 1 to 7, characterized in that each package represents a unit dose containing 0,5 to 20 mg of said first antibody or said cytotoxic conjugate.

9. Cytotoxic pharmaceutical combination according to claim 1, characterized in that it consists of an injectable solution containing both said first antibody and said cytotoxic conjugate in a mole ratio between 0,1 and 10.

10. Cytotoxic pharmaceutical combination according to claim 1, useful for the treatment of cancers, characterized by the fact that the said first antibody is a tumoral anticell antibody.

11. Cytotoxic pharmaceutical combination according to claim 10, characterized in that said first antibody is selected among the antibodies anti-T65, anti-T200, anti-HLA, and in that said cytotoxic conjugate is the coupling product of the A chain of ricin with mouse IgG.

1/1